# EUROPEAN PATENT SPECIFICATION

(11) **EP 2 856 157 B1**
(45) Date of publication and mention of the grant of the patent: **28.12.2016**
(21) Application number: 13725646.7
(22) Date of filing: 24.05.2013
(51) Int. Cl.: G01N 33/53

(54) **METHOD FOR THE DETECTION OF GLYCANS USING POLYPEPTIDES COMPRISING THE CD301 CARBOHYDRATE RECOGNITION DOMAIN**
VERFAHREN ZUM NACHWEIS VON GLYCANEN UNTER VERWENDUNG VON POLYPEPTIDEN UMFASSEND DEN CD301 KOHLENHYDRAT-ERKENNUNGSDOMÄN
PROCÉDÉ DE DÉTECTION DE GLYCANES UTILISANT DES POLYPEPTIDES COMPRENANT LE DOMAINE DE RECONNAISSANCE D'HYDRATES DE CARBONE DE CD301

(30) Priority: 28.05.2012 EP 12004109
(43) Date of publication of application: 08.04.2015
(73) Proprietor: Universitätsklinikum Hamburg-Eppendorf, 20246 Hamburg (DE)
(72) Inventor: WAGENER, Christoph, 20149 Hamburg (DE); NOLLAU, Peter, 20146 Hamburg (DE); NIENDORF, Axel, 22587 Hamburg (DE)
(74) Representative: Uexküll & Stolberg
(86) International application number: PCT/EP2013/060813
(87) International publication number: WO 2013/178559

(56) References cited:
- WO-A2-2008/007960
- SUZUKI N. ET AL.: "Molecular cloning and expression of cDNA encoding human macrophage C-type lectin: Its unique carbohydrate binding specificity for Tn antigen", J. IMMUNOL., vol. 156, no. 1, 1 January 1996 (1996-01-01), pages 128-135, XP002046614,
- WAGENER C. ET AL.: "Posttranslational modifications in tumor diagnosis", CLIN. CHEM. LAB. MED., vol. 46, no. Suppl. 1, May 2011 (2011-05), page S9, XP009164779,
- NOLLAU P. ET AL.: "Protein domain histochemistry (PDH): binding of the carbohydrate recognition domain (CRD) of recombinant human glycoreceptor CLEC10A (CD301) to formalin-fixed, paraffin-embedded breast cancer tissues", J. HISTOCHEM. CYTOCHEM., vol. 61, no. 3, March 2013 (2013-03), pages 199-205, XP9171781,
- MORTEZAI N. ET AL.: "Tumor-associated Neu5Ac-Tn and Neu5Gc-Tn antigens bind to C-type lectin CLEC10A (CD301, MGL)", GLYCOBIOLOGY, vol. 23, no. 7, July 2013 (2013-07), pages 844-852, XP55074447, [retrieved on 2013-03-18]
- JOHANSSON M.E.V. ET AL.: "The two mucus layers of colon are organized by the MUC2 mucin, whereas the outer layer is a legislator of host-microbial interactions", PROC. NAT. ACAD. SCI. USA, vol. 108, no. Suppl. 1, March 2011 (2011-03), pages 4659-4665,

## Description

### CARBOHYDRATE RECOGNITION DOMAIN

The present invention relates to a method for the detection of glycans in a tissue sample, which method comprises contacting the tissue sample with a recombinant polypeptide, said polypeptide comprising the carbohydrate recognition domain of a human CD301 polypeptide or a sequence having at least 80% sequence identity thereto, and detecting recombinant polypeptide that binds to said tissue sample. The disclosure also describes multimeric complexes comprising at least two of the above-described recombinant CD301 polypeptides and, optionally, an antibody which binds to said polypeptides.

### BACKGROUND OF THE INVENTION

Posttranslational modifications (PTMs) such as phosphorylation and glycosylation are important regulators of functional states of normal or diseased cells and tissues. For example, it has been found that certain cancer diseases are regularly associated with an altered glycan pattern that occurs on the surface of tumor or tumor-associated cells. For a number of such cancer diseases, staining procedures are available that are based on the specific detection of aberrant glycosylation, thereby allowing differentiation between normal and malignant cells in embedded tissue sections.

At present, most histological techniques which aim at staining glycans make use of glycan-binding antibodies. However, the production of commonly used antibodies is laborious and expensive. Also, for some glycan structures no antibodies are commercially available. Therefore, new and improved histological methods for the staining of glycans are needed.

The human genome encodes several proteins and protein complexes that are capable of recognizing PTMs. For example, phosphotyrosine-containing amino acid motifs are bound by SH2-domains, and cell surface glycans were found to interact with the carbohydrate recognition domains (CRD) of glycoreceptors of the C-type lectin family.

Glycoreceptors of the C-type lectin family recognize different glycan and glycoprotein structures. For example, it is known that the human glycoreceptor CD301 (CLEC10A) binds to a number of different glycan structures, among which are Tn (GalNAca), 6-Sulfo-Tn, core5, core6 and Lac-di-NAc. CD301 is expressed by intermediate monocytes (Wong et al. (2011), Blood, 118:e16-31; Ziegler-Heitbrock et al. (2010), Blood, 116:e74-80) and macrophages (van Vliet et al. (2008), Trends Immunol, 29:83-90). Tumor associated macrophages (TAMs) play an important role in tumor progression. TAMs that foster tumor progression have been reported to express CD301 (Prokop S. et al. (2011), Am J Pathol 2011, 178:1279-1286; Solinas G. et al. (2010), J Immunol, 185:642-652). Hence, it is speculated that binding of these macrophages to glycans via CD301 might play a role in tumor progression.

WO 2008/007960 discloses a method for detecting tumour cells comprising contacting a sample comprising said cells with a carbohydrate binding part of a macrophage galactose-type lectin (MGL) and detecting the presence of cells that have bound said carbohydrate binding part. The document further relates to the use of a carbohydrate binding part of MGL for the preparation of a composition for the detection of primary tumour cells of epithelial origin and to a protein comprising a carbohydrate recognition domain of MGL.It is preferred that the carbohydrate recognition domain of MGL is coupled to a multimerisation domain so as to create multimers. The carbohydrate recognition domain of MGL is located in the 141 C-terminal amino acids, i.e., amino acids 152-292.

The present invention is based on the insight that recombinant polypeptides comprising the carbohydrate binding portion of the human glycoreceptor CD301 can be used for staining glycans with high specificity in biochemical assays as well as in tissue samples. Surprisingly, the carbohydrate binding portions of CD301 are able to penetrate fixed and embedded tissue and provide sufficient binding specificity to allow a reliable detection of glycan structures.

The present invention hence provides new histochemical methods which are based on the use of portions of the human glycoreceptor CD301 as a glycan binding molecule. By use of recombinant polypeptides which include portions of the glycoreceptor CD301, the disadvantages known from the use of lectins and antibodies can be circumvented.

### DISCLOSURE OF THE INVENTION

In a first aspect, the present invention relates to a method that aims at the detection and/or staining of glycans in a paraffin-embedded cell or tissue sample. The method comprises the steps
(a) providing a paraffin-embedded cell or tissue sample;
(b) removing the paraffin from the cell or tissue sample by a solvent and re-hydrating the sample;
(c) contacting the cell or tissue sample with a recombinant, glycan-binding polypeptide that comprises the carbohydrate recognition domain (CRD) of human CD301 depicted in SEQ ID NO:1 or a sequence having at least 80% sequence identity thereto provided that the resulting variant CRD still exhibits a glycan-binding activity ;
(d) detecting whether said recombinant polypeptide binds to said cell or tissue sample,
wherein binding of said recombinant polypeptide to said cell or tissue sample indicates that the cells in the sample comprise glycans.

The above method is carried out with a cell or tissue sample that has been obtained directly from a test subject or from an isolated tissue, e.g. from a surgically removed tumor. A variety of different sample types may be used, provided that the sample contains cells that are normally expected to contain glycan residues, e.g. on their cell surface. Preferably, the sample is a tissue sample obtained from the test subject, e.g., by core needle biopsy, fine needle aspiration, and the like. The sample may contain fresh tissue, or it may contain frozen tissue material. A cell sample for use in the method of the invention may be a blood sample or a fraction (e.g. the leukocyte fraction) of a blood sample that is obtained from a subject. Before being used in the methods of the invention, the cell or tissue sample to be analyzed according to the method of the invention can be processed to render the sample eligible for subsequent histological analysis, e.g., by fixation and/embedding.

The paraffin-embedded tissue sample used in step (a) of the present invention comprises or consists of frozen tissue slices that have been sectioned by use of a microtome and subsequently mounted on a glass or plastic slide. The sample is at least in part enclosed in or surrounded by a suitable embedding material (paraffin) that has been solidified after being contacted with the tissue of interest. After hardening, the embedded cells or tissues can be cut into thin sections which are then subjected to staining.

Paraffin-embedded cell or tissue samples are prepared in a multistep procedure which comprises, as an initial step, the chemical fixation of the cells or tissues to be analyzed. For this purpose, the cells or tissues are treated with chemical fixatives that cause cross-linking of the intracellular proteins. By such crosslink-linking, the structural integrity of the cells is maintained and degradation of the cell organelles is prevented. Typically used chemical fixatives include formalin (a 4-8% formaldehyde solution) and glutaraldehyde. Samples which are to be analyzed by light microscopy are typically fixed by use of formalin. Following fixation, the fixed cells or tissue samples are dehydrated by immersing the sample, e.g. in increasing concentrations of ethanol. Then, the sample is treated with a solvent, such as TPC solvent (Instrumedics) or xylene, to remove the alcohol. Finally, the sample is treated with molten paraffin which infiltrates the sample.

After dehydration and infiltration, the cells or tissues are subjected to embedding. This means, the cells or tissues samples are contacted with liquid paraffin. Typically, the cells and tissues are placed into molds, and the paraffin, is poured into the molds. The embedding material is then hardened by cooling the embedding material. The molded blocks with the cell or tissue sample inside can be sectioned into slices, e.g. by use of a microtome. Prior to staining the sample with the recombinant CD301 polypeptide, the paraffin of the cell or sample is removed by a solvent like TPC solvent or xylene. Subsequently, the sample is re-hydrated by immersing the sample firstly into 100% ethanol followed by ethanol solutions of decreasing concentrations.

In a particularly preferred embodiment of the invention, the method of the invention uses a cell or tissue sample which has been formalin-fixed and subsequently paraffin-embedded.

One technical field which benefits from the methods provided by the present invention is the field of cancer or tumor analysis. The cell or tissue samples to be analyzed with the method of the invention are thus preferably tumor tissue samples for which it is intended to detect the particular glycan pattern. In general, the cells or tissues of the samples can be derived from all different types of solid tumors, for example, pancreatic, breast, colon, brain, prostate, lung, bronchial, liver, bladder, skin, ovarian, cervical, stomach, kidney, uterine, esophageal, laryngeal, nasopharyngeal, testicular, vulvar, salivary gland, thyroid, parathyroid, small intestine, thymus, and other tumors.

Other cancer cells that may be analyzed by the method of the present invention include hematological malignancies, in particular leukemias, such as acute lymphoblastic leukemia (ALL), acute myelogenous leukemia (AML), chronic lymphocytic leukemia (CLL), chronic myelogenous leukemia (CML), and acute monocytic leukemia (AMOL). In a still further embodiment, the tissue to be analyzed is a tissue that shows signs of chronic or acute inflammation.

It is, however, preferred that the cell or tissue samples which are analyzed by the methods of the present invention are derived from a breast, colon, pancreas, lung or bladder tumor. In a particularly preferred embodiment, the tissue is derived from a breast tumor. Important types of malignant breast tumors comprise an invasive ductal carcinoma, an invasive lobular carcinoma, a papillary carcinoma, a mucinous carcinoma, a medullary carcinoma and a tubular carcinoma. The diagnosis and classification of cancers is well known in the art and discussed in numerous publications (see, for example, Tavassoli et al. (2003), World Health Organization: Tumours of the Breast and Female Genital Organs, WHO/IARC Classification of Tumours).

The glycans to be detected according to the method of the invention are preferably glycans which have been reported to be relevant for the diagnosis of pathological conditions, and in particular cancer diseases. As disclosed in the examples of the present invention, a polypeptide comprising the CRD of human CD301 binds to the same glycan species that have been reported to interact with human CD301. Accordingly, in a preferred embodiment, the glycan to be detected can be selected from the group consisting of Tn (α-N-acetyl-D-galactosamine), Lac-di-Nac (GalNAcβ1-4GlcNAc-R), core5 (2-acetamido-2-deoxy-3-O-(2-acetamido-2-deoxy-a-O-galactopyranosyl)-α-O-galactopyranose), core6 (2-acetamido-2-deoxy-6-O-(2-acetamido-2-deoxy-β-D-glucopyranosyl)-α-D-galactopyranose, GalNAcβ (β-N-acetyl-D-galactosamine), 6-Sia-Tn (6-sialyl-Tn), α-Neu5Gc-Tn (2-acetamido-2-deoxy-6-O-(α-N-glycolylneuraminyl)-α-D-galactopyranose), 6-su-α-Tn (6-sialyl-α-Tn), Atri (A-antigen, A-trisaccharide; terminal sugars of blood group A), Fs-2 (Forssman disaccharide) and 6-su-GalNAc (α-N-acetyl-D-galactosamine-6-sulfate). It is known that the pattern of these glycans is modified in some tumor tissues compared to the corresponding normal tissues. For example, in some tumors the level of glycosylation is increased compared to normal healthy cells.

In a first step of the method of the invention, the cell or tissue sample to be analyzed is brought into contact with a recombinant polypeptide that comprises the carbohydrate recognition domain of human CD301 or a sequence having at least 80% sequence identity thereto. As used herein, the term "recombinant" refers to the fact that the polypeptide has been artificially produced, e.g. by genetic engineering, rather than isolated from its natural environment. Preferably, the recombinant polypeptide comprising the carbohydrate recognition domain of human CD301 has been obtained by heterologous expression of the polypeptide in host cells, preferably eukaryotic host cells.

Human CD301 is a glycoprotein having a molecular weight of about 40 kDa comprising a cytoplasmic domain, a transmembrane domain and an extracellular domain. The human genome contains a single gene for CD301. Alternate splicing generates two isoforms of human CD301 which differ in by certain deletions in the coiled-coiled domain. The sequences of the two different isoforms are provided herein as SEQ ID NO:5 (isoform 1) and SEQ ID NO:6 (isoform 2), respectively. The extracellular domain of CD301, which is identical in both isoforms, contains a carbohydrate recognition domain which is responsible for binding to the target glycan structures. The amino acid sequence of the extracellular domain is shown in SEQ ID NO:2, and the amino acid sequence of the carbohydrate recognition domain is depicted in SEQ ID NO:1.

In a preferred embodiment of the invention, the recombinant polypeptide comprises a CRD having the sequence provided in SEQ ID NO:1. However, as will be appreciated by the person of skill, the ability of amino acid sequence of SEQ ID NO:1 to bind to target glycan structures is likely to remain unchanged if one or more amino acids of the CRD are deleted or substituted, or if one or more amino acids added. Accordingly, the present invention also relates to methods for the detection of glycans which use recombinant polypeptides that comprise a "variant" CRD which differs from the CRD shown in SEQ ID NO:1 in one or more than one amino acid positions. Compared to the CRD depicted in SEQ ID NO:1, the sequence variants encompassed by the present invention exert at least part of the binding activity towards their target glycans. Specifically, a variant CRD is considered herein to have glycan-binding activity if it binds to a glycan selected from the group consisting of Tn, Lac-di-Nac (GalNAcβ1-4GlcNAc-R), core5, core6, GalNAcβ, 6-Sia-Tn, α-Neu5Gc-Tn, 6-su-α-Tn, Atri, Fs-2 and 6-su-GalNAc with at least 10%, more preferably 20%, 30%, 40%, 50%, 60%, 70%, 80%, 90%, 95% or more of the binding activity of the CDR of wild-type CD301 depicted in SEQ ID NO:1 as determined in a binding assay, e.g. the binding assay described below in Example 3.

In general, any amino acid residue of the sequence shown in SEQ ID NO:1 may be deleted or substituted by another amino acid, provided that the resulting variant CRD still exhibits a glycan-binding activity as defined above. Similarly, one or more amino acids may be added at any position of the sequence shown in SEQ ID NO:1 as long as this addition does not abolish the binding of the modified CRD to the glycan target structure. In particular, the methods of the invention are performed with variant CRDs which differ from the amino acid sequence shown in SEQ ID NO:1 in up to 5, 10, 15, 20, 25, or even up to 30 amino acid positions.

Where the variant CRD comprises one or more amino acid substitutions, it is preferred that these substitutions are conservative substitutions. As used herein, conservative substitutions are substitutions of an amino acid residue by another amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also encompassed by the invention are variant CRD sequences which include additional amino acids compared to the sequence of SEQ ID NO:1, i.e. CRD sequences in which one or more amino acids have been inserted relative to the wild-type CRD. Such insertions may in principle occur at any position of the CRD of SEQ ID NO:1. The insertions may be stretches of contiguous amino acids comprising, for example, 2, 3, 4, 5, 6, 7, 8, 9 or ten amino acids. Similarly, variant CRD are also meant to include CRD sequences in which one or more amino acids have been deleted compared to the CRD of SEQ ID NO:1. The deletion can involve several contiguous amino acid residues of the CRD sequence of SEQ ID NO:1, for example, 2, 3, 4, 5, 6, 7, 8, 9 or ten amino acids. Variant of the CRD of SEQ ID NO:1 may also include structural modifications, for example modified amino acids, such as amino acids that have been altered by phosphorylation, glycosylation, acetylation, thiolation, branching and/or cyclization.

Variants of the CRD sequence of SEQ ID NO:1, which have been modified by substitution, addition or deletion of one or more amino acids, will normally show a considerable degree of amino acid sequence identity to the CRD sequence of SEQ ID NO:1. Preferably, the identity on the amino acid level is at least 70%, 75%, 80%, 85%, 90%, 91%, 92%, 93%, 94%, 95%, 96%, 97%, 98% or 99% if the sequence of the variant CRD is optimally aligned with SEQ ID NO:1. Methods and computer programs for determining amino acid identity are known in the art.

Preferably, the recombinant polypeptide comprising the CRD of SEQ ID NO:1 or its variant has an overall size of less than 250 amino acids, preferably less than 200 amino acids, more preferably less than 170, 160, 150, 140, 130 and most preferably less than 120 amino acids, such as 118 amino acids. In some embodiments, the recombinant polypeptide may be a fragment of the CRD having the sequence of SEQ ID NO:1. The fragment may contain less than 118 amino acids as long as the fragment still provides for at least part of the binding characteristics of the polypeptide set forth in SEQ ID NO:1. In a particularly preferred embodiment, the recombinant polypeptide comprising the CRD of SEQ ID NO:1 or its variant is less than 150 amino acids in size and is for use in paraffin-embedded samples.

The recombinant polypeptide which comprises the glycan-binding domain of human CD301 can be produced by any method known in the art to be suitable for the production of peptides or polypeptides. In a preferred aspect, the recombinant polypeptide that comprises the CRD of human CD301 or a sequence having at least 80% sequence identity thereto has been produced by heterologous expression in a mammalian expression system. Mammalian expression systems which have been proven useful for the heterologous expression of human polypeptides include primary cells and tissues (e.g. murine bone marrow cells, haematopoietic stem cells, murine lymphocytes, muscle tissue of different species, hepatocytes, and the like) as well as cultured cells (e.g. Chinese hamster ovary (CHO) cells, monkey kidney cells (COS), baby hamster kidney (BHK) cells, immortalized murine blood lymphocytes, and the like).

Mammalian expression systems and appropriate expression constructs are widely known in the art. The expression construct can be, for example, a viral or non-viral expression vector which is useful for introducing the polynucleotides into a cell for subsequent expression of polypeptides encoded by said polynucleotides. The expression vector can be an episomal vector, i.e. one that is capable of self-replicating autonomously within the host cell, or an integrating vector, i.e. one which stably incorporates into the genome of the cell.

An expression vector will normally comprise a promoter which is functionally linked to the polynucleotide which encodes the polypeptide to be expressed. Suitable promoters include, but are not limited to, the cytomegalovirus promoter (CMV), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector may also optionally comprise transcription termination sequences and polyadenylation sequences for improved expression of the polypeptide of interest. Suitable transcription terminator and polyadenylation signals can, for example, be derived from SV40 (Sambrook et al (1989), Molecular Cloning: A Laboratory Manual).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus, adeno-associated virus (AAV), orthomyxovirus, paramyxovirus, papovavirus, picornavirus, or alphavirus. For an overview of the different viral vector systems, see Nienhuis et al., Hematology, Vol. 16: Viruses and Bone Marrow, N.S. Young (ed.), 353-414 (1993).

Retroviral vectors are particularly preferred. Retroviral vectors normally function by transducing and integrating the selected polynucleotide into the genome of the target cell. The retroviral vectors can be derived from both of the subfamilies, orthoretroviruses and spumaviruses. For example, vectors from Murine Sarcoma Virus, Bovine Leukemia, Virus Rous Sarcoma Virus, Murine Leukemia Virus, Mink-Cell Focus-Inducing Virus, Reticuloendotheliosis Virus, Avian Leukosis Virus can be used. In a particularly preferred aspect, the viral vector used for introducing the polynucleotide encoding the recombinant CD301 polypeptide or its variant into the host cell is a lentiviral vector, such as a LeGO vector (e.g. LeGO iG2). The skilled person will furthermore be able to combine portions derived from different retroviruses, such as LTRs, tRNA binding sites, and packaging signals to provide a recombinant retroviral vector. These retroviral vectors are then normally used for producing transduction competent retroviral vector particles. For this purpose, the vectors are introduced into suitable packaging cell lines, such as those described in US patent 5,591,624. Retrovirus vectors can also be constructed for site-specific integration into the DNA of the host cell by incorporating a chimeric integrase enzyme into the retroviral particle (see WO 96/37626).

Alternatively, non-viral vector systems may also be used for introducing the polynucleotide encoding the recombinant CD301 polypeptide or its variant into the host cell. Non-viral vector systems typically involve non-viral expression vectors which are introduced into the cell, e.g. by liposome-mediated transfer, direct injection of naked DNA, or the gene gun technology. A preferred vector for expressing the recombinant polypeptides of the invention is the mammalian expression vector EBB (Mizushima and Nagata (1990), Nucl Acids Res, Vol. 18, No. 17, page 5322). Another preferred vector for expressing the recombinant polypeptide is the expression vector pcDNA 3.1 (Invitrogen).

Several mammalian expression systems are available from different manufacturers and can be employed in the present invention, such as plasmid- or viral vector based systems, e.g. LENTI-Smart^{™} (InvivoGen), GenScript^{®} Expression vectors, pAd-VAntage™ (Promega), ViraPower™ Lentiviral and Adenoviral Expression Systems (Invitrogen).

Expression systems based on non-mammalian cells may also be used for producing the recombinant CD301 polypeptide. For example, the host cell may be a bacterial, insect or yeast cell. Accordingly, the expression of the recombinant polypeptide can involve commonly known expression systems which make use of such cells. For example, the recombinant polypeptide can be expressed in insect cells, e.g. in cells of *Aedes aegypti, Autographa californica, Bombyx mori, Spodop tera frugiperda* und *Trichoplusia ni,* etc., by use of a baculovirus expression system such as the Bac-to-Bac Baculovirus expression system (Invitrogen, San Diego, California, USA). Alternatively, expression can be performed in bacterial cells, for example, cells of *Bacillus subtilis,* and *Escherichia coli.* For example, the QIAgene Expression Kits for *E. coli* of Qiagen (Hilden, Germany) can be used. If expression in yeasts is desired, commercial expression systems can be used, for example, the Easy Select™ Pichia Expression Kit (Invitrogen, San Diego, California, USA). It is, however, preferred that the recombinant CD301 polypeptide to be used in the methods of the present invention has been expressed in a mammalian host cell, more preferably in a HEK293T cell line.

When contacting the tissue sample to be analyzed with the recombinant polypeptide that comprises a CRD derived from CD301, said recombinant polypeptide is preferably dissolved in an adequate buffer. The dissolved polypeptide is applied to the tissue sample by pipetting the solution containing the polypeptide onto the tissue sample, e.g. an embedded tissue slice that is mounted a glass slide or a microtiter plate. Alternatively, the tissue sample can be completely or partially immersed into the solution that contains the glycan-binding polypeptide of the invention. The amount of recombinant polypeptide that is to be added to the tissue sample depends on different factors, such as the polypeptide that is used for detection, the way of detection employed for visualizing binding of the polypeptide to the tissue sample, and the size and nature of the tissue sample. Where the cell or tissue sample is a paraffin-embedded sample mounted on a glass or plastic slide, the amount of recombinant polypeptide, e.g. the CRD opf SEQ ID NO:1, to be used for an effective detection of glycans is in the range of 0.1-50 µg per cm² of tissue surface area. Preferably, the amount of recombinant polypeptide, e.g. recombinant CD301, to be used is about 1, 5, 10, 15, 20, 25, 30, 35, 40, 45 or 50 µg per cm² of tissue surface area.

After contacting the tissue sample with the recombinant polypeptide, the sample will be incubated for 2-48 h, preferably 8-12 h at 4-20°C, and preferably 4-8°C, to allow for binding of the polypeptides to their target structure. This incubation step can be performed in a wet chamber. Following incubation, the binding of the polypeptides of the invention to the glycans in the cell or tissue sample will be detected. Numerous techniques for the detection of polypeptides that are bound to cells or tissues are known in the art and can be employed in the methods of the present invention. Such techniques will usually comprise detection of a marker that is directly or indirectly (e.g. via an antibody that specifically binds to the recombinant polypeptide) associated with the recombinant polypeptide. A suitable marker for use in the methods of the invention can be any marker which is detectable by analytical means and which confers this property to the recombinant polypeptide. The term "marker" as used herein can comprise single detectable molecules as well as sets of two or more molecules, such as the Biotin/Streptavidin system.

Suitable marker molecules include, e.g., fluorescent molecules or luminescent molecules with absorbance spectra that can be monitored for detection. Numerous markers are known to those skilled in the art and are suitable for use with the present invention. The CRD-containing polypeptides of the present invention may, for example, be coupled to enzymes such as horseradish peroxidase or alkaline phosphatase, or recognition sequences for enzymes (such as the BirA biotinylation), or Biotin. In one embodiment, the recombinant polypeptide is directly coupled to biotin and subsequently detected via the high affinity binding of biotin to avidin or streptavidin, or to modified streptavidin. In such an approach, the avidin or streptavidin may be labelled with an enzyme such as horseradish peroxidase or alkaline phosphatase.

The marker may also be indirectly linked to the polypeptides used in the methods of the present invention. For example, specific binding of the marker to the recombinant polypeptides for use in the methods of the invention may be mediated e.g. by antibody binding, i.e. the marker is e.g. coupled to an antibody which specifically binds to the recombinant polypeptide. In one embodiment, the marker biotin is covalently linked to another molecule, preferably an antibody, which specifically recognizes and binds to the recombinant polypeptide. In such an embodiment, the recombinant CD301 polypeptide can be contacted with the tissue sample, and after the polypeptide has bound to the tissue sample, the biotin-labelled antibody is contacted with the complex consisting of the glycan-binding polypeptide and its target glycan structure. Alternatively, the polypeptide may first be contacted with the antibody, and the resulting immune complex is then contacted with the sample. The biotin-labelled antibody which has bound to the complex consisting of the glycan-binding polypeptide and its target glycan structure can then be cross-linked with other antibodies, for example by avidin or streptavidin labelled with an enzyme such as horseradish peroxidase or alkaline phosphatase. Alternatively, cross-linking can be achieved by use of secondary antibodies that bind to the antibodies which are directed to the CRD-containing recombinant polypeptides. In this setup, the secondary antibodies will be labelled with one of the markers discussed above, preferably with horseradish peroxidase or alkaline phosphatase.

In a further aspect of the invention, the recombinant polypeptide may be expressed as a fusion polypeptide that comprises one or more detectable sequence tags. Such fusion polypeptides can be detected via an antibody or antibody fragment that is directed to the tag or by a material which has a particularly high affinity for the sequence tag. Suitable amino acid tags comprise, e.g. a His-tag with 6-12 histidine residues, a myc-tag, maltose binding protein (MBP), a glutathione-S-transferase-tag, or a streptag. The person skilled in the art will be able to identify suitable detection means for each of these tags. For example, labelled antibodies that are specific for each of these tags are commercially available. In a preferred embodiment, the recombinant polypeptide of the invention is labelled with a myc-tag. In an even more preferred embodiment, the recombinant polypeptide of the invention is labelled with a myc-tag and a His-tag.

In a particularly preferred aspect, the recombinant glycan-binding polypeptide used in the method of the invention is present in the form of a multimeric complex. The multimeric complex comprises at least two recombinant polypeptides, each comprising the carbohydrate recognition domain of SEQ ID NO:1 or a sequence having at least 80% sequence identity thereto. Optionally, the complex may also comprise an antibody which binds to said recombinant polypeptides (or their tag sequences). The avidity of the multimeric complex is higher than that of the single polypeptide and, thus, provides an improved sensitivity in comparison with the single polypeptide.

The multimeric complex may comprise at least two or more identical recombinant CD301 polypeptides or different CD301 polypeptides. Thus, different variants of the carbohydrate recognition domain of a human CD301 depicted in SEQ ID NO:1 or a sequence having at least 80% sequence identity thereto may be comprised in the same multimeric complex.

The at least two recombinant polypeptides are covalently or non-covalently bound within the multimeric complex. The polypeptides will be bound within the multimeric complex in a manner that facilitates that the binding sites of the at least two recombinant polypeptides are exposed such that each is able to bind to the glycan target structures.

For example, the at least two recombinant polypeptides may be covalently linked with each other, either directly or by one or more bridge molecules. A "bridge molecule" as used herein may be any molecule that allows a sterically favourable position of the at least two recombinant polypeptides in the multimeric complex. For example, the one or more bridge molecules may be amino acid linker sequences of a suitable length or non-proteinogenic, chemical crosslinkers.

A suitable amino acid linker sequence has a length of between 3 and 50 amino acids, between 7 and 50 amino acids, between 4 and 40 amino acids, between 5 and 30 amino acids, between 6 and 20 amino acids, between 7 and 10 amino acids, or between 7 and 25 amino acids. In particular, the length of the linker can be 4, 5, 6, 7, 8, 9, 10, 11, 12, 13, 14, 15, 16, 17, 18, 19, 20, 21, 22, 23, 24 or 25 amino acids. Amino acid linker sequences are known in the art and can be derived, for example, from HIV gp41, the human immunoglobulin G (IgG), preferably IgG2, the human P-glycoprotein, the human replication protein A, or parathyroid hormone-related proteins.

Alternatively, the at least two recombinant polypeptides may be bound within the multimeric complex by a bifunctional crosslinker that connects two recombinant polypeptides with each other. However, when more than two recombinant polypeptides are bound in the multimeric complex, the crosslinker may have more than two binding sites for the recombinant polypeptides. Suitable non-proteinogenic, chemical crosslinkers include, e.g. the imidoester crosslinker dimethyl suberimidate, the N-hydroxysuccinimide-ester crosslinker BS3 or formaldehyde. Suitable for multimerization are also streptavidin-coated beads, for example, magnetic or sepharose beads. Suitable beads are for example Strep-Tactin Magnetic nanobeads (IBA BioTAGnology, IBA GmbH, Germany). Several other suitable bridge molecules are known in the art.

In another embodiment, the CRD-containing recombinant polypeptides in the multimeric complexes comprise biotin. The attachment of biotin can be achieved by common methods which are well known in the art. For example, biotin can be attached to polypeptides by chemical coupling or by in-vivo biotinylation, using e.g. a BirA ligase which attaches a biotin group to a lysine residue of the polypeptide. The antibodies which carry one or more biotin groups can then be multimerized by avidin or streptavidin. For visualization of the complexes, avidin or streptavidin can be labelled with a marker that allows the detection of the complex. As explained above, suitable markers comprise fluorescent markers, radioactive markers and enzymes, such as horseradish peroxidase or alkaline phosphatase.

When the at least two recombinant polypeptides are non-covalently bound within the multimeric complex, the strength of the binding must be sufficient to enable the multimeric complex to remain substantially intact during the course of the method of the present invention.

In a preferred embodiment, the at least two recombinant polypeptides are bound in the multimeric complex by an antibody that binds to said recombinant polypeptides. The at least two recombinant polypeptides may be bound in the multimeric complex by one ore more antibodies, i.e. a sufficient number of antibodies to bind all recombinant polypeptides into the complex. The antibody binds specifically to the polypeptides, for example to an amino acid tag sequence comprised in the recombinant polypeptides or to any other part of the polypeptide which does not interfere with binding of the CRD sequence to its target glycan structures. A suitable amino acid tag sequence may be inter alia any of those described above, preferably a myc-tag. Other suitable tag sequences include an HA-tag, a Flag-tag, a BiRa-tag, and an E-tag. The recombinant polypeptides within the multimeric complex of the invention may also include more than one of the same type of tag which are consecutively fused to the CRD-containing recombinant polypeptides. For example, more than one myc-tag may be fused to the polypeptides, e.g. 2-5 myc-tags. If necessary, the antibodies may be linked to each other or to a third molecule. In this manner, the multimeric complex can increase considerably in size and avidity.

In one embodiment, the multimeric complex comprising the CRD-containing recombinant polypeptides comprises at least two antibodies, for example at least 3, 4, 5, 6, 7, 8, 9 or 10 antibodies which may be cross-linked with each other, e.g. by streptavidin or by use of secondary antibodies. The antibodies within the multimeric complex may or may not be labelled with a marker as explained above.

In one embodiment two recombinant polypeptides are non-covalently bound to a single antibody. In a more preferred embodiment the multimeric complex comprises two recombinant polypeptides, each comprising the CRD of CD301 according to SEQ ID NO:1 or a variant thereof and a myc-tag, wherein the polypeptides are bound by a single anti-myc antibody. In a particularly preferred embodiment, the antibody is covalently coupled to biotin, so that cross-linking of said antibody with other antibodies is feasible via avidin or streptavidin. As described in the below examples, avidin or streptavidin can be labelled with an enzyme for visualization of the complexes.

Accordingly, in still another aspect, the invention therefore relates to a multimeric complex, said complex comprising
(a) at least two recombinant polypeptides as described above, each comprising the carbohydrate recognition domain of human CD301 depicted in SEQ ID NO:1 or a sequence having at least 80% sequence identity thereto;
(b) an antibody which binds to said recombinant polypeptides.

Preferably, the said recombinant polypeptide within the multimeric complex comprises a myc-tag and said antibody is an anti-myc-antibody. More preferably, the anti-myc-antibody comprises streptavidin.

In still another aspect, the invention relates to a recombinant polypeptide comprising a carbohydrate recognition domain of a human CD301 depicted in SEQ ID NO:1 or a sequence having at least 80% sequence identity thereto and a detectable marker.

The invention also relates to the use of a multimeric complex as described above or a recombinant polypeptide as described herein for the detection or staining of glycans in a tissue sample.

### BRIEF DESCRIPTION OF THE FIGURES

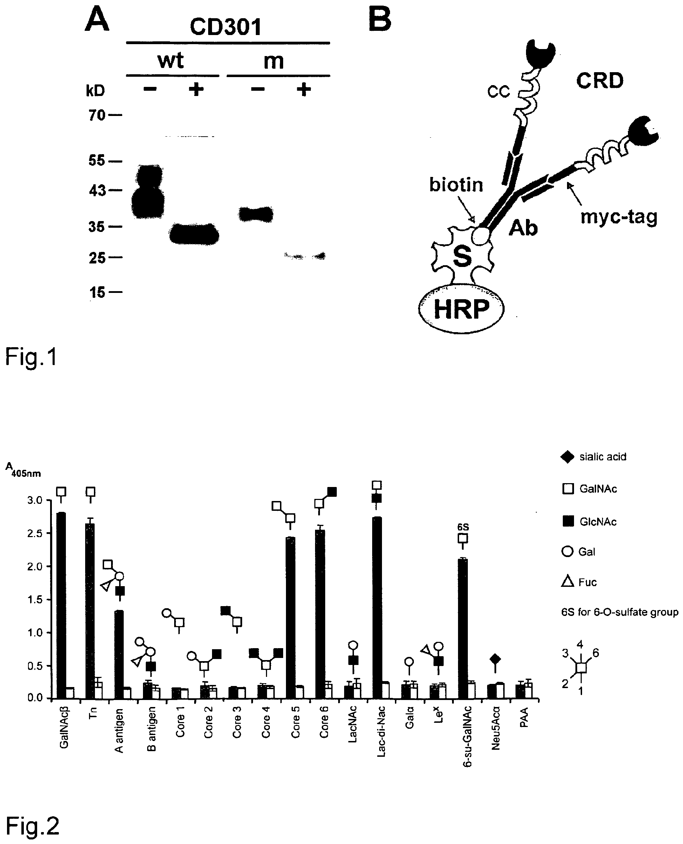

Fig. 1 (A) shows the results from the Western Blot analysis of CD301 probe (wt) and CD301 mutant probe (m) expressed in tissue culture supernatants of HEK293T cells. Soluble polypeptides were detected applying the anti-myc antibody 9E10 and a HRP-conjugated polyclonal rabbit anti-mouse serum followed by ECL. For validation of polypeptide binding, native preparations (-) were compared to CD301 preparations after deglycosylation (+).
Fig. 1 (B) shows the structure of the multimeric CD301 complex formed between CD301 probe, the biotinylated anti-myc antibody 9E10 recognizing the myc-tag at the N-terminus of CD301 probe and horseradish peroxidase (HRP) conjugated streptavidin (S). The extracellular part of CD301 is composed of the carbohydrate recognition domain (CRD) followed by a coiled-coiled (cc) domain.

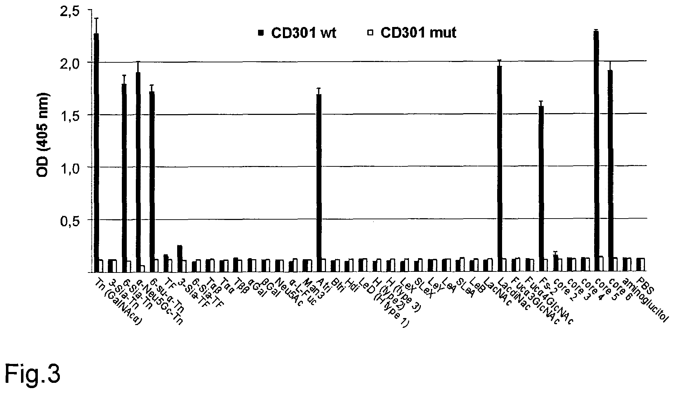

Fig. 2 and 3 show the functional characterization and determination of binding specificity of CD301 probe and Cd301 mutant probe determined by a glycan ELISA with different immobilized glycoconjugates. The composition of glycans conjugated to polyacrylamide (PAA) are given by the pictograms. Measurements were performed in triplicate; error bars indicate the standard error of the mean. PAA served as background control.

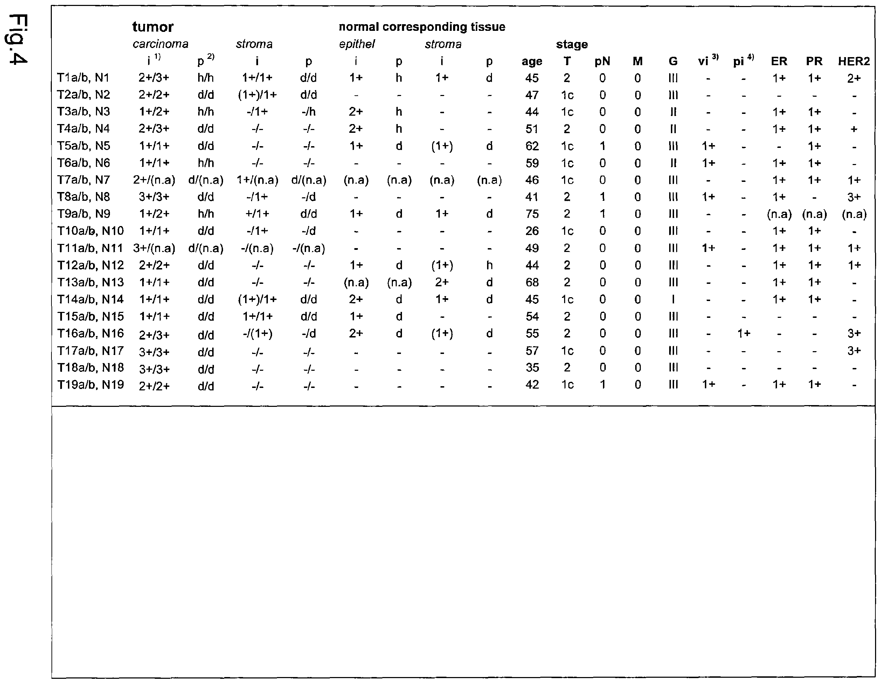

Fig. 4 shows the staining intensity and staining pattern of CD301 probe that was contacted with 19 human breast cancer specimens and normal tissue. CD301 probe staining was performed on a tissue microarray composed of tumor tissue samples in duplicate (n = 2x 19 represented by a and b) and a single, corresponding normal tissue sample for each patient (TXa/b, NX; X = number of specimen). Tissue spots T7b, T11b and N7 were lost during processing. Staging is in accordance to the AJCC classification 2010. Abbreviations: ¹⁾ i = staining intensity: 1+ = weak, 2+ = moderate, 3+ = strong; ²⁾ s = staining pattern: d = diffuse, h = heterogeneous; grading (G) I = well, II = moderately and III = poorly differentiated according to the Bloom-Richardson classification; ³⁾ vi = vascular invasion; ⁴⁾ pi = perineural invasion; ER / PR = estrogen /progesterone receptor and HER2 = erbB2/NEU receptor staining; (-) = absent, (n.a.) = not available.

### EXAMPLES

### EXAMPLE 1: Cloning and expression of recombinant CD301-CRD

A recombinant polypeptide which includes the extracellular domain of the human CD301 was constructed by genetic engineering. In a first step, the extracellular domain of human CD301 isoform 2 was amplified by PCR from a human cDNA library (Clontech, CA, USA). The PCR primers shown in SEQ ID NO:7 (5'-CGGATATCAGGGACCTGGTGACCCTGAGAACAG-3') and SEQ ID NO:8 (5'-TCGGTACCTCAGTGACTCTCCTGGCTGGTCTGACC-3') were used in the following PCR program:

| | |
|---|---|
| initial: | 95°C - 5 min |
| 35 cycles: | 95°C - 30 sec |
| | 69°C - 45 sec |
| | 72°C - 3 min |
| final: | 72°C - 10 min |

The resulting PCR product was cloned into the eukaryotic EBB expression vector that was previously described in Bogoevska V. et al. (2006), Glycobiology, 16:197-209. The cloned extracellular domain comprised amino acids 67-292 of the full-length CD301. The sequence of the cloned extracellular domain is shown in SEQ ID NO:2, and the CRD which is located within the extracellular domain is depicted in SEQ ID NO:1. To enforce secretion into the tissue culture supernatant, a human IgGκ-leader was attached at the N-terminus followed by a His-tag and a myc-tag for CD301 complexing and detection. The recombinant polypeptide was designated "CD301 probe". The sequence of CD301 probe is shown in SEQ ID NO:3.

To obtain a non-binding negative control, a mutant of CD301 probe was generated by truncation of 57 aa from the C-terminus. Thereby, approximately 60% of the C-terminal part of the carbohydrate recognition domain was removed. Like CD301 probe, the mutant included a human IgGκ-leader sequence at the N-terminus followed by a His-tag and a myc-tag. The recombinant polypeptide was designated "CD301 mutant probe". The sequence of CD301 mutant probe is shown in SEQ ID NO:4.

The cloned plasmid-DNA constructs were transiently transfected into HEK293T cells by transfection with Lipofectamine^{™} 2000 (Invitrogen, Karlsruhe, Germany). Tissue culture supernatants were harvested after 48h and cleared by centrifugation to investigate recombinant protein expression of CD301 probe and CD301 mutant probe. Expression of recombinant CD301 probe and CD301 mutant probe was characterized by Western Blot analysis in comparison with an internal standard for quantification as previously described in Bogoevska V. et al. (2006), Glycobiology, 16:197-209. Supernatants were either directly used or stored at 4°C in the presence of 0.01% sodium azide for further analysis.

Soluble, recombinant CD301 probe and CD301 mutant probe were harvested from tissue culture supernatants and analyzed by Western Blot analysis via the myc-tag (Figure 1A). CD301 probe migrated as a broad band with a molecular weight of 35 kD to 50 kD, while CD301 probe was detectable with an apparent molecular weight of approximately 35 kD. The migratory behavior of the recombinant proteins differs from the calculated theoretical molecular weights with 30 kD for CD301 probe and 24 kD for CD301 mutant probe, respectively, indicating that both proteins are posttranslationally modified, most likely by glycosylation during the expression in HEK293T cells.

For determination of the exact molecular weight, glycans were removed from the probes applying the protein deglycosylation mix (NEB, Frankfurt, Germany). Deglycosylation of CD301 probe and CD301 mutant probe resulted in a shift in electrophoretic mobility to the expected molecular weights, confirming the identity and size of the recombinant probes (Figure 1A).

### EXAMPLE 2: Multimerization of recombinant CD301 probe

To increase the binding strength of the glycoreceptor probe, the avidity was increased by multimerization of CD301 domains. For this purpose, CD301 probe and CD301 mutant probe were bound to a biotinylated anti-myc antibody via their myc-tags (Figure 1B). 100-200 ng/ml of recombinant CD301 probe and mutant probe, respectively, as estimated from Western Blot analysis in comparison to an internal standard (data not shown) were incubated with 2 µg/ml of biotinylated anti-myc antibody 9E10 (Santa Cruz Biotechnology, CA, USA) for 1h at 4°C. Multimerization was achieved by complexing the CD301-antibody complex via biotin to streptavidin conjugated to horseradish-peroxidase (HRP) (Thermo Fisher Scientific/Pierce, IL, USA) for detection (Figure 1B). Streptavidin-HRP conjugate was added to a final concentration of 2.5 µg/ml and incubated for 1h at 4°C.

### Example 3: Functionality and binding specificity of multimerized probes

To test the functionality and binding specificity of the multimerized probes, a glycan-based ELISA was applied. Glycoconjugates were purchased from Lectinity (Moscow, Russia) composed of a poly[N-(2-hydroxyethyl)acrylamide] (PAA) backbone to which different carbohydrates were linked via aminoglucitol (HOCH₂(HOCH)₄CH₂NH-X). Different PAA-conjugated glycans (1 µg/well) were immobilized to flat-bottom MaxiSorp 96 well plates (Thermo Fisher Scientific/Nunc, IL, USA) in 100 µl PBS (pH 7.4) at 4°C over night under constant agitation. Plates were blocked using 1x carbo-free blocking solution (Biozol, Eching, Germany) in distilled water for 1 hour at room temperature. Unbound glycoconjugates were removed by washing three times.

CD301 probe and C301 mutant probe complexes were freshly prepared as described in Example 2. 100 µl of complex were added per well and incubated for 2h at room temperature. Three washing steps with TSM-buffer (20 mM Tris/HCl pH 7.4, 150 mM NaCl, 2 mM MgCl₂, 1 mM CaCl₂) in the presence of 0.1% Tween 20 were carried out. To be able to photometrically determine binding of the complexes, ABTS (2,2'-Azino-di-[3-ethylbenzthiazoline sulfonate (6)] diammonium salt) was added as a substrate according to the instructions of the manufacturer (Roche, Mannheim, Germany). Absorbance was measured at 405 nm on a microplate reader (Tecan, Maennedorf, Switzerland) at 37°C.

The wild-type CRD of CD301 probe strongly bound to terminal GalNAc glycostructures present in the Tn-antigen, blood group A, the Lac-di-NAc sequence (GalNAcβ1-4GlcNAc-R), 6-su-GalNAc as well as core 5 and core 6 structures (Figure 2, dark bars). No binding was observed for the CD301 mutant probe, confirming that truncation of the CRD completely abrogates the binding of the mutated CRD (Figure 2, light bars). Since CD301 mutant probe contains a non-functional CRD, ligand binding can be assigned to the CRD of CD301 probe. The observed binding preferences for CD301 are in good accordance with data on human CD301 (MGL) published by van Vliet S.J. et al. (2005), Int Immunol, 17:661-669.

### Example 4: CD301 binding to in carcinoma and normal tissue

Having confirmed the functionality and specificity of the probes, CD301 probe and CD301 mutant probe multimers were applied for staining of paraffin-embedded breast cancer specimen (n=19) and normal corresponding tissue arranged on a tissue microarray (TMA). Paraffin embedded TMAs of formalin fixed human breast cancer and corresponding normal tissue were purchased from BioCat (Heidelberg, Germany). Tissue sections were deparaffinized in xylol (2x 5 min each) and rehydrated in PBS. Antigen retrieval was achieved by boiling (5x 2 min each) in a microwave (900 W) in 0.1 M sodium citrate buffer (pH 5.0). Slides were washed in PBS, endogenous peroxidase activity was blocked by 3% hydrogen peroxide for 5 min at room temperature and slides were washed once in TSM buffer (20 mM Tris/HCl pH 7.4; 150 mM NaCl; 2 mM MgCl₂, 1 mM CaCl₂). Subsequently, unspecific binding sites were blocked at 4°C for 2h in TSM-buffer in the presence of 0.2% BSA, 10% fetal calf serum and 0.3% Triton X-100.

During the blocking step, a complex of myc-tagged CD301 probe, streptavidin-HRP conjugate and the biotinylated anti-myc antibody 9E10 was freshly prepared as described in Example 2. Tissue sections were covered with 200 µl of complex and incubated at 4°C overnight in a wet chamber. After washing (3x 5 min each) in TSM-buffer, staining was performed with 3,3'-diamino-benzidine chromogen solution (Dako, Hamburg, Germany) for 10 min at room temperature, slides were washed in H₂O once and nuclei were counterstained by hematoxylin. The stained tissue section was mounted applying Glycergel Mounting Medium (Dako). Microscopic examination was performed with an Axiphot microscope (Zeiss, Jena, Germany) and pictures were taken with a AxioCam (MRc5; Zeiss) camera at a magnification of 150-fold. Tissue sections were independently reviewed and scored by two pathologists. Fisher's exact test was applied for statistical analysis.

CD301 staining in carcinoma and corresponding normal tissue was evaluated with regard to intensity and distribution. Additionally, patterns of distribution were assigned to normal tissue, the parenchyma and stroma of carcinomas as shown in Figure 3. The staining patterns in the parenchyma of tumors were diffuse in most of the carcinomas; in 4 of 19 cases a heterogeneous pattern of staining was observed. Tumor stroma stained almost negative with very faint and sporadic staining of single fibroblasts.

Likewise and occasionally, inflammatory cells present in the stroma of tumors were found to stain positive. Normal epithelial cells scored weakly or moderately positive and staining was primarily localized to lobular epithelial cells and was accentuated to the luminal part of the acinus. Connective tissues of the normal parenchyma and fat cells stained completely negative. In a single case weak staining of endothelial cells in normal and cancerous tissue was observed. No staining was observed for the CD301 mutant probe emphasizing the high binding specificity of CD301 probe comprising the wild-type CRD. In addition, intensity and patterns of staining matched very well between duplicates of tumor samples of the same patient demonstrating that staining with CD301 multimers is highly specific and well reproducible.

Positive staining with CD301 probe complexes was observed in all carcinomas, with strong staining intensity in approximately two-thirds of the samples (13/19). In contrast, CD301 staining was absent or weak in 13 of 17 normal breast epithelia. These results indicate that CD301 staining is a suitable marker for the assessment of a carcinoma risk.

Although the number of carcinoma specimens investigated in this study was small, associations between staining intensity and histopathological as well as clinical parameters were evaluated. Interestingly, CD301 staining intensity of carcinomas significantly correlated (p < 0.004) with positivity for expression of the tumor marker HER2/neu receptor.

## Claims

1. Method for the detection and/or staining of glycans in a paraffin-embedded cell or tissue sample, comprising
(a) providing a paraffin-embedded cell or tissue sample;
(b) removing the paraffin from the cell or tissue sample by a solvent and re-hydrating the sample;
(c) contacting the cell or tissue sample with a recombinant polypeptide, said polypeptide comprising the carbohydrate recognition domain (CRD) of human CD301 depicted in SEQ ID NO:1 or a sequence having at least 80% sequence identity thereto;
provided that the resulting variant CRD still exhibits a glycan-binding activity;
(d) detecting whether said recombinant polypeptide binds to said cell or tissue sample,
wherein binding of said recombinant polypeptide to said paraffin-embedded cell or tissue sample indicates that the cells in the sample comprise glycans.

2. Method according to claim 1, wherein said paraffin-embedded cell or tissue sample is a tumor tissue sample.

3. Method according to claim 2, wherein said paraffin-embedded cell or tissue sample is derived from a breast, colon, pancreas, lung or bladder tumor.

4. Method according to claims 1-3, wherein said glycan is selected from the group consisting of Tn-antigen, Lac-di-Nac (GalNAcβ1-4GlcNAc-R), core5, core6, GalNAcβ, 6-Sia-Tn, α-Neu5Gc-Tn, 6-su-α-Tn, Atri, Fs-2 and 6-su-GalNAc.

5. Method according to claims 1-4, wherein said polypeptide comprises one or more detectable markers which are covalently coupled to the polypeptide.

6. Method according to claims 1-5, wherein said polypeptide is a fusion polypeptide that comprises one or more detectable amino acid tags.

7. Method according to claim 6, wherein said polypeptide comprises a myc-tag.

8. Method according to claims 1-7, wherein said polypeptide is present in the form of a multimeric complex, said complex comprising
(a) at least two recombinant polypeptides, each comprising the carbohydrate recognition domain of a human CD301 depicted in SEQ ID NO:1 or a sequence having at least 80% sequence identity thereto;
(b) optionally, an antibody which binds to said recombinant polypeptides.

9. Method according to claim 8, wherein said recombinant polypeptides comprise a myc-tag and said antibody is an anti-myc-antibody.

10. Method according to claim 9, wherein said anti-myc-antibody comprises biotin.

11. Use of a recombinant polypeptide comprising the carbohydrate recognition domain (CRD) of human CD301 depicted in SEQ ID NO:1 or a sequence having at least 80% sequence identity thereto provided that the resulting variant CRD still exhibits a glycan-binding activity for the detection or staining of glycans in a paraffin-embedded cell or tissue sample.

## Patentansprüche

1. Verfahren zur Detektion und/oder Färbung von Glykanen in einer in Paraffin eingebetteten Zell- oder Gewebeprobe, bei dem man:
(a) eine in Paraffin eingebettete Zell- oder Gewebeprobe bereitstellt;
(b) das Paraffin von der Zell- oder Gewebeprobe mit einem Lösungsmittel entfernt und die Probe re-hydriert;
(c) die Zell- oder Gewebeprobe mit einem rekombinanten Polypeptid in Kontakt bringt, wobei das Polypeptid die Kohlenhydrat-Erkennungsdomäne (carbohydrate recognition domain, CRD) des humanen CD301 aufweist, welche in SEQ ID NO:1 gezeigt ist, oder eine Sequenz, die mindestens 80% Sequenzidentität zu dieser aufweist, vorausgesetzt, dass die CDR-Variante immer noch eine Glykan-bindende Aktivität aufweist;
(d) detektiert, ob das rekombinante Polypeptid an die Zell- oder Gewebeprobe bindet,
wobei eine Bindung des rekombinanten Polypeptids an die in Paraffin eingebettete Zell- oder Gewebeprobe anzeigt, dass die Zellen in der Probe Glykane umfassen.

2. Verfahren gemäß Anspruch 1, wobei die in Paraffin eingebettete Zell- oder Gewebeprobe eine Tumorgewebeprobe ist.

3. Verfahren gemäß Anspruch 2, wobei die in Paraffin eingebettete Zell- oder Gewebeprobe von einem Brust-, Kolon-, Pankreas-, Lungen-, oder Blasentumor stammt.

4. Verfahren gemäß einem der Ansprüche 1-3, wobei das Glykan ausgewählt ist aus der Gruppe bestehend aus Tn-Antigen, Lac-di-Nac (GalNAcß1-4GlcNAc-R), core5, core6, GalNAcß, 6-Sia-Tn, α-Neu5Gc-Tn, 6-su-α-Tn, Atri, Fs-2 und 6-su-GalNac.

5. Verfahren gemäß einem der Ansprüche 1-4, wobei das Polypeptid einen oder mehrere nachweisbare Marker aufweist, welche kovalent an das Polypeptid gekoppelt sind.

6. Verfahren gemäß einem der Ansprüche 1-5, wobei das Polypeptid ein Fusionspolypeptid ist, welches einen oder mehrere nachweisbare Aminosäure-Tags aufweist.

7. Verfahren gemäß Anspruch 6, wobei das Polypeptid einen Myc-Tag aufweist.

8. Verfahren gemäß einem der Ansprüche 1-7, bei dem das Polypeptid in Form eines multimeren Komplexes vorliegt, wobei der Komplex Folgendes umfasst:
(a) mindestens zwei rekombinante Polypeptide, wobei jedes davon die Kohlenhydrat-Erkennungsdomäne des humanen CD301 aufweist, welche in SEQ ID NO:1 gezeigt ist, oder eine Sequenz, die mindestens 80% Sequenzidentität zu dieser aufweist;
(b) wahlweise einen Antikörper, der an die rekombinanten Polypeptide bindet.

9. Verfahren gemäß Anspruch 8, wobei die rekombinanten Polypeptide einen myc-Tag umfassen, und der Antikörper ein Anti-myc-Antikörper ist.

10. Verfahren gemäß Anspruch 9, wobei der Anti-myc-Antikörper Biotin umfasst.

11. Verwendung eines rekombinanten Polypeptids, das die Kohlenhydrat-Erkennungsdomäne (CRD) des humanen CD301 aufweist, welche in SEQ ID NO:1 gezeigt ist, oder eine Sequenz, die mindestens 80% Sequenzidentität zu dieser aufweist, vorausgesetzt, dass die CDR-Variante immer noch eine Glykan-bindende Aktivität aufweist, zur Detektion und/oder Färbung von Glykanen in einer in Paraffin eingebetteten Zell- oder Gewebeprobe.

## Revendications

1. Procédé de détection et/ou coloration de glycanes dans un échantillon de cellules ou de tissu inclus dans de la paraffine, lequel procédé comporte les étapes suivantes :
a) prendre un échantillon de cellules ou de tissu inclus dans de la paraffine ;
b) retirer la paraffine de l'échantillon de cellules ou de tissu, à l'aide d'un solvant, er réhydrater l'échantillon ;
c) mettre l'échantillon de cellules ou de tissu en contact avec un polypeptide recombinant, lequel polypeptide comporte le domaine CRD de reconnaissance des glucides de CD301 humain, représenté en tant que Séquence N° 1, ou une séquence identique à celle-ci à un degré d'au moins 80 %, sous réserve que la variante de CRD résultante présente encore une activité de liaison aux glycanes ;
d) et détecter si ledit peptide recombinant se lie audit échantillon de cellules ou de tissu ;
étant entendu que le fait que ledit peptide recombinant se lie audit échantillon de cellules ou de tissu inclus dans de la paraffine indique que les cellules de l'échantillon contiennent des glycanes.

2. Procédé conforme à la revendication 1, dans lequel ledit échantillon de cellules ou de tissu inclus dans de la paraffine est un échantillon de tissu de tumeur.

3. Procédé conforme à la revendication 2, dans lequel ledit échantillon de cellules ou de tissu inclus dans de la paraffine provient d'une tumeur du sein, du côlon, du pancréas, du poumon ou de la vessie.

4. Procédé conforme aux revendications 1 à 3, dans lequel ledit glycane est choisi dans l'ensemble formé par les suivants : antigène Tn, Lac-di-Nac (GalNAcβ1-4GlcNAc-R), core5, core6, GalNAcβ, 6-Sia-Tn, α-Neu5Gc-Tn, 6-su-α-Tn, Atri, Fs-2 et 6-su-GalNAc.

5. Procédé conforme aux revendications 1 à 4, dans lequel ledit polypeptide comporte un ou plusieurs marqueur(s) détectable(s), qui est ou sont couplé(s) au polypeptide par liaison de covalence.

6. Procédé conforme aux revendications 1 à 5, dans lequel ledit polypeptide est un polypeptide de fusion comportant une ou plusieurs étiquette(s) détectable(s) faites d'acides aminés.

7. Procédé conforme à la revendication 6, dans lequel ledit polypeptide comporte une étiquette myc.

8. Procédé conforme aux revendications 1 à 7, dans lequel ledit polypeptide se présente sous forme de complexe multimère, lequel complexe comprend :
a) au moins deux polypeptides recombinants qui comportent chacun le domaine de reconnaissance des glucides de CD301 humain, représenté en tant que Séquence N° 1, ou une séquence identique à celle-ci à un degré d'au moins 80 % ;
b) et en option, un anticorps qui se lie auxdits polypeptides recombinants.

9. Procédé conforme à la revendication 8, dans lequel lesdits polypeptides recombinants comportent une étiquette myc et ledit anticorps est un anticorps anti-myc.

10. Procédé conforme à la revendication 9, dans lequel ledit anticorps anti-myc comporte de la biotine.

11. Utilisation d'un polypeptide recombinant, comportant le domaine CRD de reconnaissance des glucides de CD301 humain, représenté en tant que Séquence N° 1, ou une séquence identique à celle-ci à un degré d'au moins 80 %, sous réserve que la variante de CRD résultante présente encore une activité de liaison aux glycanes, pour la détection ou la coloration de glycanes dans un échantillon de cellules ou de tissu inclus dans de la paraffine.


### SEQUENCE LISTING

<110> Universitatsklinikum Hamburg-Eppendorf
<120> METHODS FOR GLYCAN STAINING USING POLYPEPTIDES COMPRISING THE CD301 CARBOHYDRATE RECOGNITION DOMAIN
<130> P 91244
<160> 8
<170> PatentIn version 3.3
<210> 1
   <211> 118
   <212> PRT
   <213> Homo sapiens
<400> 1
   His Gln Asp Ser Cys Tyr Trp Phe Ser His Ser Gly Met Ser Trp Ala
   1               5                   10                  15
   Glu Ala Glu Lys Tyr Cys Gln Leu Lys Asn Ala His Leu Val Val Ile
               20                  25                  30
   Asn Ser Arg Glu Glu Gln Asn Phe Val Gln Lys Tyr Leu Gly Ser Ala
           35                  40                  45
   Tyr Thr Trp Met Gly Leu Ser Asp Pro Glu Gly Ala Trp Lys Trp Val
       50                  55                  60
   Asp Gly Thr Asp Tyr Ala Thr Gly Phe Gln Asn Trp Lys Pro Gly Gln
   65                  70                  75                  80
   Pro Asp Asp Trp Gln Gly His Gly Leu Gly Gly Gly Glu Asp Cys Ala
                   85                  90                  95
   His Phe His Pro Asp Gly Arg Trp Asn Asp Asp Val Cys Gln Arg Pro
               100                 105                 110
   Tyr His Trp Val Cys Glu
           115
<210> 2
   <211> 226
   <212> PRT
   <213> Homo sapiens
<400> 2
   Arg Asp Leu Val Thr Leu Arg Thr Asp Phe Ser Asn Phe Thr Ser Asn
   1               5                   10                  15
   Thr Val Ala Glu Ile Gln Ala Leu Thr Ser Gln Gly Ser Ser Leu Glu
               20                  25                  30
   Glu Thr Ile Ala Ser Leu Lys Ala Glu Val Glu Gly Phe Lys Gln Glu
           35                  40                  45
   Arg Gln Ala Val His Ser Glu Met Leu Leu Arg Val Gln Gln Leu Val
       50                  55                  60
   Gln Asp Leu Lys Lys Leu Thr Cys Gln Val Ala Thr Leu Asn Asn Asn
   65                  70                  75                  80
   Gly Glu Glu Ala Ser Thr Glu Gly Thr Cys Cys Pro Val Asn Trp Val
                   85                  90                  95
   Glu His Gln Asp Ser Cys Tyr Trp Phe Ser His Ser Gly Met Ser Trp
               100                 105                 110
   Ala Glu Ala Glu Lys Tyr Cys Gln Leu Lys Asn Ala His Leu Val Val
           115                 120                 125
   Ile Asn Ser Arg Glu Glu Gln Asn Phe Val Gln Lys Tyr Leu Gly Ser
       130                 135                 140
   Ala Tyr Thr Trp Met Gly Leu Ser Asp Pro Glu Gly Ala Trp Lys Trp
   145                 150                 155                 160
   Val Asp Gly Thr Asp Tyr Ala Thr Gly Phe Gln Asn Trp Lys Pro Gly
                   165                 170                 175
   Gln Pro Asp Asp Trp Gln Gly His Gly Leu Gly Gly Gly Glu Asp Cys
               180                 185                 190
   Ala His Phe His Pro Asp Gly Arg Trp Asn Asp Asp Val Cys Gln Arg
           195                 200                 205
   Pro Tyr His Trp Val Cys Glu Ala Gly Leu Gly Gln Thr Ser Gln Glu
       210                 215                 220
   Ser His
   225
<210> 3
   <211> 268
   <212> PRT
   <213> artificial
<220>
   <223> artificial fusion protein
<400> 3
   Met Glu Thr Asp Thr Leu Leu Leu Trp Val Leu Leu Leu Trp Val Pro
   1 5 10 15
   Gly Ser Thr Gly His His His His His His Glu Gln Lys Leu Ile Ser
   20 25 30
   Glu Glu Asp Leu Gly Gly Gly Ser Asp Ile Arg Asp Leu Val Thr Leu
   35 40 45
   Arg Thr Asp Phe Ser Asn Phe Thr Ser Asn Thr Val Ala Glu Ile Gln
   50 55 60
   Ala Leu Thr Ser Gln Gly Ser Ser Leu Glu Glu Thr Ile Ala Ser Leu
   65 70 75 80
   Lys Ala Glu Val Glu Gly Phe Lys Gln Glu Arg Gln Ala Val His Ser
   85 90 95
   Glu Met Leu Leu Arg Val Gln Gln Leu Val Gln Asp Leu Lys Lys Leu
   100 105 110
   Thr Cys Gln Val Ala Thr Leu Asn Asn Asn Gly Glu Glu Ala Ser Thr
   115 120 125
   Glu Gly Thr Cys Cys Pro Val Asn Trp Val Glu His Gln Asp Ser Cys
   130 135 140
   Tyr Trp Phe Ser His Ser Gly Met Ser Trp Ala Glu Ala Glu Lys Tyr
   145 150 155 160
   Cys Gln Leu Lys Asn Ala His Leu Val Val Ile Asn Ser Arg Glu Glu
   165 170 175
   Gln Asn Phe Val Gln Lys Tyr Leu Gly Ser Ala Tyr Thr Trp Met Gly
   180 185 190
   Leu Ser Asp Pro Glu Gly Ala Trp Lys Trp Val Asp Gly Thr Asp Tyr
   195 200 205
   Ala Thr Gly Phe Gln Asn Trp Lys Pro Gly Gln Pro Asp Asp Trp Gln
   210 215 220
   Gly His Gly Leu Gly Gly Gly Glu Asp Cys Ala His Phe His Pro Asp
   225 230 235 240
   Gly Arg Trp Asn Asp Asp Val Cys Gln Arg Pro Tyr His Trp Val Cys
                   245                 250                 255
   Glu Ala Gly Leu Gly Gln Thr Ser Gln Glu Ser His
               260                 265
<210> 4
   <211> 211
   <212> PRT
   <213> artificial
<220>
   <223> CD301 fusion protein deleted (negative control)
<400> 4
   Met Glu Thr Asp Thr Leu Leu Leu Trp Val Leu Leu Leu Trp Val Pro
   1               5                   10                  15
   Gly Ser Thr Gly His His His His His His Glu Gln Lys Leu Ile Ser
               20                  25                  30
   Glu Glu Asp Leu Gly Gly Gly Ser Asp Ile Arg Asp Leu Val Thr Leu
           35                  40                  45
   Arg Thr Asp Phe Ser Asn Phe Thr Ser Asn Thr Val Ala Glu Ile Gln
       50                  55                  60
   Ala Leu Thr Ser Gln Gly Ser Ser Leu Glu Glu Thr Ile Ala Ser Leu
   65                  70                  75                  80
   Lys Ala Glu Val Glu Gly Phe Lys Gln Glu Arg Gln Ala Val His Ser
                   85                  90                  95
   Glu Met Leu Leu Arg Val Gln Gln Leu Val Gln Asp Leu Lys Lys Leu
               100                 105                 110
   Thr Cys Gln Val Ala Thr Leu Asn Asn Asn Gly Glu Glu Ala Ser Thr
           115                 120                 125
   Glu Gly Thr Cys Cys Pro Val Asn Trp Val Glu His Gln Asp Ser Cys
       130                 135                 140
   Tyr Trp Phe Ser His Ser Gly Met Ser Trp Ala Glu Ala Glu Lys Tyr
   145                 150                 155                 160
   Cys Gln Leu Lys Asn Ala His Leu Val Val Ile Asn Ser Arg Glu Glu
                   165                 170                 175
   Gln Asn Phe Val Gln Lys Tyr Leu Gly Ser Ala Tyr Thr Trp Met Gly
               180                 185                 190
   Leu Ser Asp Pro Glu Gly Ala Trp Lys Trp Val Asp Gly Thr Asp Tyr
           195                 200                 205
   Ala Thr Gly
       210
<210> 5
   <211> 316
   <212> PRT
   <213> Homo sapiens
<400> 5
   Met Thr Arg Thr Tyr Glu Asn Phe Gln Tyr Leu Glu Asn Lys Val Lys
   1               5                   10                  15
   Val Gln Gly Phe Lys Asn Gly Pro Leu Pro Leu Gln Ser Leu Leu Gln
               20                  25                  30
   Arg Leu Cys Ser Gly Pro Cys His Leu Leu Leu Ser Leu Gly Leu Gly
           35                  40                  45
   Leu Leu Leu Leu Val Ile Ile Cys Val Val Gly Phe Gln Asn Ser Lys
       50                  55                  60
   Phe Gln Arg Asp Leu Val Thr Leu Arg Thr Asp Phe Ser Asn Phe Thr
   65                  70                  75                  80
   Ser Asn Thr Val Ala Glu Ile Gln Ala Leu Thr Ser Gln Gly Ser Ser
                   85                  90                  95
   Leu Glu Glu Thr Ile Ala Ser Leu Lys Ala Glu Val Glu Gly Phe Lys
               100                 105                 110
   Gln Glu Arg Gln Ala Gly Val Ser Glu Leu Gln Glu His Thr Thr Gln
           115                 120                 125
   Lys Ala His Leu Gly His Cys Pro His Cys Pro Ser Val Cys Val Pro
       130                 135                 140
   Val His Ser Glu Met Leu Leu Arg Val Gln Gln Leu Val Gln Asp Leu
   145                 150                 155                 160
   Lys Lys Leu Thr Cys Gln Val Ala Thr Leu Asn Asn Asn Ala Ser Thr
                   165                 170                 175
   Glu Gly Thr Cys Cys Pro Val Asn Trp Val Glu His Gln Asp Ser Cys
   180 185 190
   Tyr Trp Phe Ser His Ser Gly Met Ser Trp Ala Glu Ala Glu Lys Tyr
   195 200 205
   Cys Gln Leu Lys Asn Ala His Leu Val Val Ile Asn Ser Arg Glu Glu
   210 215 220
   Gln Asn Phe Val Gln Lys Tyr Leu Gly Ser Ala Tyr Thr Trp Met Gly
   225 230 235 240
   Leu Ser Asp Pro Glu Gly Ala Trp Lys Trp Val Asp Gly Thr Asp Tyr
   245 250 255
   Ala Thr Gly Phe Gln Asn Trp Lys Pro Gly Gln Pro Asp Asp Trp Gln
   260 265 270
   Gly His Gly Leu Gly Gly Gly Glu Asp Cys Ala His Phe His Pro Asp
   275 280 285
   Gly Arg Trp Asn Asp Asp Val Cys Gln Arg Pro Tyr His Trp Val Cys
   290 295 300
   Glu Ala Gly Leu Gly Gln Thr Ser Gln Glu Ser His
   305 310 315
<210> 6
   <211> 292
   <212> PRT
   <213> Homo sapiens
<400> 6
   Met Thr Arg Thr Tyr Glu Asn Phe Gln Tyr Leu Glu Asn Lys Val Lys
   1               5                   10                  15
   Val Gln Gly Phe Lys Asn Gly Pro Leu Pro Leu Gln Ser Leu Leu Gln
               20                  25                  30
   Arg Leu Cys Ser Gly Pro Cys His Leu Leu Leu Ser Leu Gly Leu Gly
           35                  40                  45
   Leu Leu Leu Leu Val Ile Ile Cys Val Val Gly Phe Gln Asn Ser Lys
       50                  55                  60
   Phe Gln Arg Asp Leu Val Thr Leu Arg Thr Asp Phe Ser Asn Phe Thr
   65                  70                  75                  80
   Ser Asn Thr Val Ala Glu Ile Gln Ala Leu Thr Ser Gln Gly Ser Ser
                   85                  90                  95
   Leu Glu Glu Thr Ile Ala Ser Leu Lys Ala Glu Val Glu Gly Phe Lys
               100                 105                 110
   Gln Glu Arg Gln Ala Val His Ser Glu Met Leu Leu Arg Val Gln Gln
           115                 120                 125
   Leu Val Gln Asp Leu Lys Lys Leu Thr Cys Gln Val Ala Thr Leu Asn
       130                 135                 140
   Asn Asn Gly Glu Glu Ala Ser Thr Glu Gly Thr Cys Cys Pro Val Asn
   145                 150                 155                 160
   Trp Val Glu His Gln Asp Ser Cys Tyr Trp Phe Ser His Ser Gly Met
                   165                 170                 175
   Ser Trp Ala Glu Ala Glu Lys Tyr Cys Gln Leu Lys Asn Ala His Leu
               180                 185                 190
   Val Val Ile Asn Ser Arg Glu Glu Gln Asn Phe Val Gln Lys Tyr Leu
           195                 200                 205
   Gly Ser Ala Tyr Thr Trp Met Gly Leu Ser Asp Pro Glu Gly Ala Trp
       210                 215                 220
   Lys Trp Val Asp Gly Thr Asp Tyr Ala Thr Gly Phe Gln Asn Trp Lys
   225                 230                 235                 240
   Pro Gly Gln Pro Asp Asp Trp Gln Gly His Gly Leu Gly Gly Gly Glu
                   245                 250                 255
   Asp Cys Ala His Phe His Pro Asp Gly Arg Trp Asn Asp Asp Val Cys
               260                 265                 270
   Gln Arg Pro Tyr His Trp Val Cys Glu Ala Gly Leu Gly Gln Thr Ser
           275                 280                 285
   Gln Glu Ser His
       290
<210> 7
   <211> 33
   <212> DNA
   <213> artificial
<220>
   <223> PCR primer
<400> 7
   cggatatcag ggacctggtg accctgagaa cag 33
<210> 8
   <211> 35
   <212> DNA
   <213> Artificial
<220>
   <223> PCR primer
<400> 8
   tcggtacctc agtgactctc ctggctggtc tgacc 35